# EUROPEAN PATENT APPLICATION

(11) **EP 4 467 197 A2**
(43) Date of publication of application: **27.11.2024**
(21) Application number: 24192629.4
(22) Date of filing: 18.04.2019
(51) Int. Cl.: A61P 31/00

(54) **TREATMENT OF SEPSIS AND SEPTIC SHOCK**

(30) Priority: 20.04.2018 EP 18168436
(62) Divisional of application: 19717944.3
(71) Applicant: Combioxin SA, 1066 Epalinges (CH)
(72) Inventor: AZEREDO DA SILVEIRA LAJAUNIAS, Samareh, 1206 Geneva (CH); LAJAUNIAS, Frédéric, 1206 Geneva (CH)
(74) Representative: Latscha Schöllhorn Partner AG

(57) **Abstract**

The present invention relates to a composition comprising a mixture of empty liposomes, wherein said mixture of empty liposomes comprises (a) a first empty liposome comprising cholesterol, wherein the amount of cholesterol is at least 30% (weight per weight); and (b) a second empty liposome comprising sphingomyelin, for use in the treatment of sepsis, severe sepsis, septic shock, or prolonged and severe hypotension, preferably persistent hypotension, for use in the treatment of hypotension, preferably said persistent hypotension, in septic shock, sepsis, severe sepsis, acute respiratory distress syndrome or acute lung injury, or for use in the treatment of toxic shock syndrome in an animal, preferably in a human.

## Description

The present invention relates to a composition comprising a mixture of empty liposomes, wherein said mixture of empty liposomes comprises (a) a first empty liposome comprising cholesterol, wherein the amount of cholesterol is at least 30% (weight per weight); and (b) a second empty liposome comprising sphingomyelin, for use in the treatment of sepsis, severe sepsis, septic shock, or prolonged and severe hypotension, preferably persistent hypotension, for use in the treatment of hypotension, preferably persistent hypotension, in septic shock, sepsis, severe sepsis, acute respiratory distress syndrome or acute lung injury, or for use in the treatment of toxic shock syndrome in an animal, preferably in a human. In particular, the present invention relates to compositions for use in treatment of sepsis or septic shock in an animal, preferably in a human.

### RELATED ART

Sepsis is a potentially life-threatening organ dysfunction caused by a dysregulated host response to infection. It is characterized by physiologic, pathologic, and biochemical abnormalities leading to organ dysfunction (Levy M et al. Intensive Care Med 2003; 29:530-38; Singer M et al. JAMA. 2016; 315(8):801-810). Sepsis may result in tissue damage, multisystem organ failure and, subsequently, death (Cohen, 2002, Nature 420, 885-891). Approximately 30 to 50% of sepsis patients still die despite of maximal care. The mechanisms underlying this systemic dysregulated inflammatory response are complex and may involve multiple pathways. Sepsis can therefore not be effectively treated by anti-microbials alone. Indeed, despite the availability of antibiotics, current therapies to treat sepsis have proven ineffective. Neutralization of specific inflammatory cytokines have also failed, highlighting the need for new treatments (Wenzel and Edmond, 2012, N Engl J Med 366, 2122-2124).

Septic shock occurs in a subset of patients suffering from sepsis and is associated with increased mortality. The pathophysiology of septic shock is not precisely understood; it comprises underlying circulatory and cellular/metabolic abnormalities are profound enough to substantially increase mortality. Patients with septic shock can be identified with a clinical construct of sepsis with persisting hypotension requiring vasopressors to maintain a mean arterial pressure of 65 mm Hg or higher, inadequate organ perfusion, and a serum lactate level greater than 2 mmol/L (18 mg/dL) despite adequate volume resuscitation (Singer M et al. JAMA 2016; 315(8):801-10).

Sepsis and septic shock have lasting effects on patients. For example, prolonged tissue hypoperfusion can lead to long-term neurologic and cognitive sequelae.

In addition to bacterial infections, viral infections, by increasing susceptibility to bacterial co-infection, predispose patients to suffer from infections that may cause sepsis or septic shock. For instance, influenza patients frequently display increased susceptibility to *Streptococcus pneumoniae* co-infection and sepsis has been reported as the prevalent cause of mortality during influenza pandemics. The detailed mechanisms by which viral infections predispose patients to bacterial infection and ensuing sepsis are not fully understood, and treatments to treat and prevent sepsis and septic shock in patients suffering from viral infections are also needed.

Tailored empty liposomes such as empty liposomes composed of cholesterol and/or sphingomyelin and their use for the treatment of bacterial infections have recently been described to serve as traps for virulence factors such as bacterial toxins, enzymes and virulent appendages (WO 2013/186286; Henry BD et al., Nat Biotechnol 2015; 33(1):81-88; Azeredo da Silveira, S and Perez, A, Expert Rev. Anti Infect. Ther. 2015; 13(5):531-533; Azeredo da Silveira, S. and Perez, A. Expert Rev Anti Infect Ther 2017; 15:973-975). These tailored empty liposomes have also been described to display antiviral activity and, thus, as a treatment against viral infection, in particular, as a treatment neutralizing enveloped virus such as influenza virus (WO 2017/216282).

### SUMMARY OF THE INVENTION

The preferred composition of the present invention showed surprisingly positive results improving clinical signs and symptoms in a first-in-man study with patients suffering from severe pneumonia. Moreover and importantly, it has been surprisingly found that said preferred composition of the present invention resulted in improved hemodynamic parameters, prevention of hemodynamic deterioration, and faster resolution of septic shock. As a consequence, the compositions of the present invention were effective in treating sepsis and septic shock as revealed by a faster normalization of hemodynamic instability leading to patient's cure and faster discharge from the intensive care unit (ICU). Based on this promising efficacy data and its believed mechanism of action, efficacy in a envisaged further study with patients suffering from a suspected or confirmed infection regardless of the pathogen, and presenting signs of development of complications or severity, notably suffering from a community-acquired pneumonia, hospital-acquired pneumonia, ventilation-associated pneumonia, intra-abdominal infections, skin and soft tissue infections, urinary tract infections, or bacteraemia is therefore plausible. Further, based on this promising efficacy data and its believed mechanism of action, the inventive compositions are in particular believed to be highly beneficial for treating and preventing sepsis or septic shock caused by or associated with bacterial or viral pathogens that use specific lipid microdomains to attack the host and thus the animal, preferably the human patient.

Thus, in a first aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of sepsis, severe sepsis, septic shock, or prolonged and severe hypotension, preferably persistent hypotension, for use in the treatment of hypotension, preferably persistent hypotension, in septic shock, sepsis, severe sepsis, acute respiratory distress syndrome or acute lung injury, or for use in the treatment of toxic shock syndrome in an animal, preferably in a human.

In another aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of sepsis, severe sepsis, septic shock, or prolonged and severe hypotension, preferably persistent hypotension, in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of septic shock in an animal, preferably in a human.

Further aspects and embodiments of the present invention will become apparent as this description continues.

### DESCRIPTION OF FIGURES

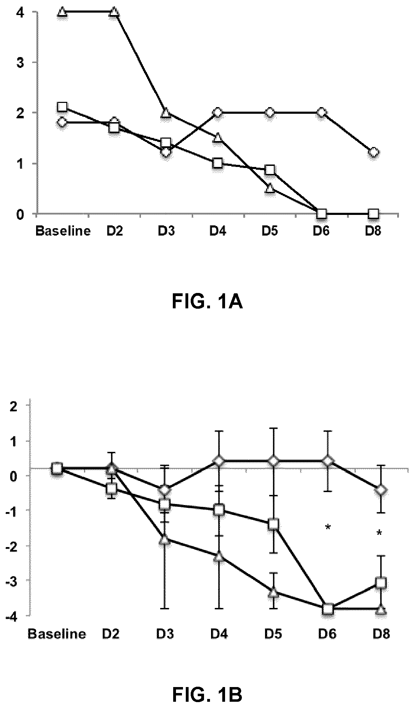

- FIG. 1:: Evolution of the Cardiovascular SOFA score in the whole study population presented in absolute values (FIG. 1A), and in the whole study population excluding patients without any hypotension event (three patients in the CAL02 High Dose arm) presented as score difference from baseline (FIG. 1B), from baseline (pre-dose) to Day 8 in the Placebo (diamonds), CAL02 Low Dose (triangles) and CAL02 High Dose (squares) groups. * p<0.05

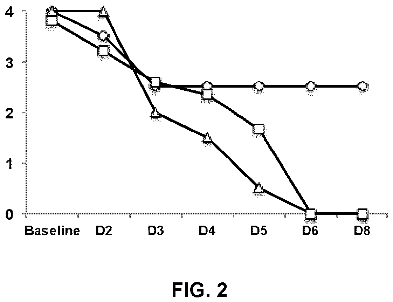

- FIG. 2:: Evolution of the Cardiovascular SOFA score in patients that were already in septic shock at baseline, presented in absolute values, from baseline (pre-dose) to Day 8 in the Placebo (diamonds), CAL02 Low Dose (triangles) and CAL02 High Dose (squares) groups.

### DETAILED DESCRIPTION OF THE INVENTION

Unless defined otherwise, all technical and scientific terms used herein have the same meanings as commonly understood by one of ordinary skill in the art to which this invention belongs.

The term "about", as used herein shall have the meaning of +/- 5%. For example about 50% shall mean 47.5% to 52.5%. Preferably, the term "about", as used herein shall have the meaning of +/- 3%. For example about 50% shall mean 48.5% to 51.5%.

When the terms "a," or "an" are used herein, they mean "at least one" unless indicated otherwise. In particular, the use of the terms "a," or "an" in association with said single empty liposome, said first empty liposome and said second empty liposome to describe the empty liposomes and mixtures of empty liposomes in accordance with the present invention should typically and preferably refer to single empty liposomes and mixtures of empty liposomes comprising said first empty liposomes and said second empty liposomes.

All ranges of values disclosed herein, should refer to all values falling within said range including the values defining the range. By way of clarification, for example, a value of 12 to 13 should refer to a value of 12 or 13 or all values falling within 12 and 13.

The term "empty liposome" as used herein, refers to liposomes, preferably artificial liposomes, having a mean diameter of 20 nm to 10 µm, preferably of 20 to 500 nm, and further preferably having a mean diameter of 20 nm to 400 nm, again further preferably of 40 nm to 400 nm or 20 nm to 200 nm, and consist of one or more phospholipid bilayers, and are typically and preferably unilamellar vesicles and multilamellar vesicles, more preferably small unilamellar vesicles (SUVs). In a preferred embodiment, the term "empty liposome" as used herein, typically and preferably refers to liposomes not incorporating any drug, typically and preferably to liposomes not incorporating any pharmaceutical drug. "Incorporated/Incorporating" as used herein and when referring to the inventive empty liposomes, typically and preferably, means encapsulated/encapsulating into the cavity of the liposome, within the potential double layer of the liposome, or as part of the membrane layer of the liposome. In another preferred embodiment, the term "empty liposome" as used herein, typically and preferably refers to liposomes consisting of sphingomyelin and cholesterol or consisting of sphingomyelin in accordance with the present invention and solely further comprises water-soluble inorganic compounds and/or water-soluble organic molecules, wherein typically and preferably said water-soluble inorganic compounds and/or water-soluble organic molecules derive from the synthesis of said inventive empty liposomes, and wherein typically and preferably, said water-soluble inorganic compounds are inorganic salts preferably selected from NaCl, KCl, MgCl₂, and wherein said water-soluble organic molecules are buffering agents, wherein preferably said water-soluble organic molecules are selected from glucose and HEPES. Typically and preferably, said water-soluble inorganic compounds and/or water-soluble organic molecules are incorporated in the inventive empty liposomes of the present invention due to their presence during the production of the inventive empty liposomes. In another preferred embodiment, the term "empty liposome" as used herein, typically and preferably refers to liposomes consisting of sphingomyelin and cholesterol or consisting of sphingomyelin in accordance with the present invention and wherein said empty liposomes do not comprise an antioxidant. In a further preferred embodiment, the term "empty liposome" as used herein, typically and preferably refers to liposomes consisting of sphingomyelin and cholesterol or consisting of sphingomyelin in accordance with the present invention and solely further comprises water-soluble inorganic compounds and/or water-soluble organic molecules, wherein typically and preferably said water-soluble inorganic compounds and/or water-soluble organic molecules derive from the synthesis of said inventive empty liposomes, and wherein typically and preferably, said water-soluble inorganic compounds are inorganic salts preferably selected from NaCl, KCl, MgCl₂, and wherein said water-soluble organic molecules are buffering agents, wherein preferably said water-soluble organic molecules are selected from glucose and HEPES, and wherein said empty liposomes consisting of sphingomyelin and cholesterol or consisting of sphingomyelin in accordance with the present invention do not comprise an antioxidant.

Sepsis or septic shock: Sepsis is a disease which has infectious cause and which shows the pathology of systemic inflammatory response syndrome (SIRS); it is defined as life-threatening organ dysfunction caused by a dysregulated host response to infection (Levy M et al. Intensive Care Med 2003; 29:530-38; Singer M et al. JAMA. 2016; 315(8):801-810). Initial symptoms found include ague, sweating, fever, and decrease in the blood pressure. When various inflammatory mediators and blood coagulation factors increase in the whole body, disturbance in the microcirculation occurs, and this results in the worsening of the pathological conditions; septic shock includes organ perfusion abnormalities, uncontrolled hypotension, and multiple organ dysfunction, which can result in death. Clinical construct of sepsis with persisting hypotension requiring vasopressors to maintain MAP ≥65 mm Hg and having a serum lactate level >2 mmol/L (18 mg/dL) despite adequate volume resuscitation. Thus, the term "sepsis" as used herein should refer to a life-threatening organ dysfunction caused by a dysregulated host response to infection, as defined and recommended in Singer M et al. JAMA. 2016; 315(8):801-810 (Recommendations; Box 3). Organ dysfunction can be identified as an acute change in total SOFA (Sequential [Sepsis-related] Organ Failure Assessment) score ≥2 points consequent to the infection. The baseline SOFA score can be assumed to be zero in patients not known to have preexisting organ dysfunction. A SOFA score ≥2 reflects an overall mortality risk of approximately 10% in a general hospital population with suspected infection. Even patients presenting with modest dysfunction can deteriorate further, emphasizing the seriousness of this condition and the need for prompt and appropriate intervention, if not already being instituted. Patients with suspected infection who are likely to have a prolonged ICU stay or to die in the hospital can be promptly identified as being more likely to have poor outcomes typical of sepsis if they have at least 2 of the following clinical criteria that together constitute a new bedside clinical score termed quick SOFA (qSOFA): respiratory rate of 22/min or greater, altered mentation, i.e., alteration in mental status, or systolic blood pressure of 100 mm Hg or less. The term "septic shock" as used herein, and as defined and recommended in Singer M et al. JAMA. 2016; 315(8):801-810 (Recommendations; Box 3) is a subset of sepsis in which underlying circulatory and cellular/metabolic abnormalities are profound enough to substantially increase mortality, and thus septic shock are associated with a greater risk of mortality than with sepsis alone. Patients with septic shock can be identified with a clinical construct of sepsis with persisting hypotension requiring vasopressors to maintain mean arterial pressure (MAP) ≥65 mm Hg and having a serum lactate level >2 mmol/L (18mg/dL) despite adequate volume resuscitation (and thus in the absence of hypovolemia). With these criteria, hospital mortality is in excess of 40%.

Animal: The term "animal" as used herein refers to aliving multi-cellular vertebrate organisms, a category that includes, for example, mammals and birds. The term mammal includes both human and non-human mammals. Similarly, the term "subject" includes both human and veterinary subjects.

The term "treating", "treatment" or "therapy" as used herein refers to means of obtaining a desired physiological effect. The effect may be therapeutic in terms of partially or completely curing a disease or a condition and/or symptoms attributed to the disease or the condition including ameliorating a sign or symptom of a disease or pathological condition related to the disease, such as sepsis or septic shock, such as reducing fever or stabilizing blood pressure in a subject with septic shock or ameliorating symptoms such as, but not limited to, ague, sweating or increasing organ function. The term "treating" as used herein in its broadest sense, should include and refer to "prevention" of a disease. "Preventing" or "prevention" of a disease refers to inhibiting the disease or condition, such as sepsis or septic shock, i.e. arresting its partial or full development of a disease, such as sepsis or septic shock, such as and for example in a person with a bacterial infection or at risk of a bacterial infection. The term "treating" as used herein in its preferred sense, thus, for the sake of its definition and to characterize preferred aspects and embodiments of the present invention, should exclude and should not refer to "prevention" of a disease. In turn, in other embodiments and aspects of the present invention of preventing, in particular, sepsis or septic shock, in an animal, preferably in a human who is at risk of sepsis or septic shock, the compositions for use and the methods of the present invention are then utilized to delay or prevent the development of sepsis or septic shock. Thus, the method includes selecting a human patient at risk for sepsis or septic shock typically and preferably caused by an infection, and administering to the human patient one or more of the compositions disclosed herein. The human patient may be, for example, someone intubated i.e. under invasive mechanical ventilation, or having been exposed to a particular bacteria, such as *Streptococcus pneumoniae* or *Staphylococcus aureus.*

Patients likely to have sepsis are well described in Singer et al. JAMA 2016: any 2 of 3 clinical variables - Glasgow Coma Scale score of 13 or less, systolic blood pressure of 100 mm Hg or less, and respiratory rate 22/min or greater -offer predictive validity similar to that of the full SOFA score outside the ICU. This model proved robust to multiple sensitivity analyses including a more simple assessment of altered mentation (Glasgow Coma Scale score <15) and in the out-of-hospital, emergency department, and ward settings within the external US and non-US data sets. For patients with suspected infection within the ICU, the SOFA score has predictive validity superior to that of this model, likely reflecting the modifying effects of interventions (eg, vasopressors, sedative agents, mechanical ventilation).

A "therapeutically effective amount" is a quantity of a composition to achieve a desired effect in a subject being treated. For instance, this can be the amount necessary to inhibit septic shock, reduce fever, or prevent multi-organ failure in an animal, preferably in a human patient, for example in a pneumonia patient and/or infected with *Streptococcus pneumoniae.* When administered to an animal, preferably to a human patient, a dosage will generally be used that will achieve effective target tissue concentrations.

A "therapeutic dose" as used herein refers to a dose which is known to the skilled person in the art to have a therapeutic effect.

The term "pneumonia" as used herein should encompass "Community Acquired Pneumonia" (CAP), "Hospital Acquired Pneumonia" (HAP) or "Ventilator-associated pneumonia" (VAP).

The term "Community Acquired Pneumonia" or "CAP" is known to the person skilled in the art, see e.g. the IDSA/ATS Guidelines for CAP in Adults (CID 2007:44 (Suppl 2) S27). In particular, the term refers to pneumonia acquired outside the hospital.

The term "Hospital Acquired Pneumonia (HAP)" refers to pneumonia acquired during or after hospitalization for another illness or procedure with onset at least 48 to 72 hours after admission.

As defined herewith, "ventilator associated pneumonia (VAP)" is a pneumonia that develops 48 hours or longer after mechanical ventilation and that is characterized by an invasion of the lower respiratory tract and lung parenchyma by microorganisms. VAP is a potentially serious medical condition.

Pneumonia is caused by infection with a broad range of microorganisms including bacteria such as *Streptococcus pneumoniae, Haemophilus influenzae, Legionella pneumophilia, Staphylococcus aureus* and *Pseudomonas aeruginosa* for CAP and gram-negative bacilli such as *Pseudomonas aeruginosa* and *Serratia marcescens,* as well as *Staphylococcus aureus, Klebsiella pneumoniae, Escherichia coli, Stenotrophomonas maltophilia, Acinetobacter species,* and *Haemophilus influenzae* for HAP (cf. Cilloniz et al., Thorax. 2011 Apr;66(4):340-6 and Jones RN. Clin InfectDis 2010 Aug; 51(Suppl1):S81-7).

The term "severe Community Acquired Pneumonia" or "sCAP" is known to the skilled person. In particular, the term "severe Community Acquired Pneumonia" or "sCAP" refers to the subgroup of Community Acquired Pneumonia patients that require intensive care. The Infectious Disease Society of America (IDSA) and the American Thoracic Society (ATS) have issued guidelines on the management of CAP including a definition of sCAP (cf. Mandell et al., 2007, Infectious Diseases Society of America/American Thoracic Society Consensus Guidelines on the Management of Community-Acquired Pneumonia in Adults, Clin. Inf. Dis. 2007:44:S27-72 (Suppl 2), Table 4). According to the IDSA/ATS guidelines sCAP is defined as CAP requiring intensive care. Admission to the intensive care unit is recommended if the CAP patient shows one or both of two major criteria, or presence of three minor criteria from the list as described in Example 1 and as effected in the presented study.

As used herein, the term "for use" as used in "composition for use in treatment of a disease" shall disclose also the corresponding method of treatment and the corresponding use of a preparation for the manufacture of a medicament for the treatment of a disease".

In one aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in treatment of septic shock in an animal, preferably in a human,

In another aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in treatment of hypotension, preferably persistent hypotension, in an animal, preferably in a human, wherein said hypotension, preferably said persistent hypotension, is related to a septic shock.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of sepsis, severe sepsis, septic shock, or prolonged and severe hypotension preferably persistent hypotension, for use in the treatment of hypotension, preferably persistent hypotension, in septic shock, sepsis, severe sepsis, acute respiratory distress syndrome or acute lung injury, or for use in the treatment of toxic shock syndrome in an animal, preferably in a human.

In another aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of sepsis, severe sepsis, septic shock, or prolonged and severe hypotension preferably persistent hypotension, in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of sepsis or severe sepsis in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of septic shock in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of prolonged and severe hypotension preferably persistent hypotension, in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of hypotension, preferably persistent hypotension, in septic shock, sepsis, severe sepsis, acute respiratory distress syndrome or acute lung injury in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of persistent hypotension in septic shock in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of hypotension, preferably persistent hypotension, in septic shock in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of persistent hypotension in septic shock in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of hypotension, preferably persistent hypotension, in sepsis or severe sepsis in an animal, preferably in a human.

In a further aspect, the present invention provides for a composition comprising, preferably consisting of, a mixture of empty liposomes, wherein said mixture of empty liposomes comprises, preferably consists of, (a) a first empty liposome comprising cholesterol, wherein the amount of cholesterol is at least 30% (weight per weight); and (b) a second empty liposome comprising sphingomyelin; for use in the treatment of toxic shock syndrome in an animal, preferably in a human.

In a further aspect and very preferred embodiment, the present invention provides said inventive composition for use in the treatment of hypotension, preferably persistent hypotension, of said animal, preferably said human, with sepsis. In a further aspect and very preferred embodiment, the present invention provides said inventive composition for use in the treatment of hypotension, preferably persistent hypotension, of said human, with sepsis. In a further aspect and very preferred embodiment, the present invention provides said inventive composition for use in the treatment of persistent hypotension, of said human, with sepsis.

In a further aspect and very preferred embodiment, the present invention provides said inventive composition for use in the treatment of hypotension, preferably persistent hypotension, of said animal, preferably said human, with septic shock. In a further aspect and very preferred embodiment, the present invention provides said inventive composition for use in the treatment of hypotension, preferably persistent hypotension, of said human, with septic shock. In a further aspect and very preferred embodiment, the present invention provides said inventive composition for use in the treatment of persistent hypotension, of said human, with septic shock.

In another aspect, the invention provides for a method of treating sepsis or septic shock, preferably septic shock in an animal, preferably in a human in need thereof, the method comprising administering a therapeutically effective amount of a composition as defined in the appended claims. Preferably, said human patient has pneumococcal pneumonia or said sepsis or septic shock, preferably septic shock, results from pneumococcal pneumonia.

For all aspects and embodiments disclosed herein, a therapeutically effective amount of the inventive compositions are typically and preferably used for the disclosed treatments.

Moreover, all embodiments and preferred embodiments as disclosed herein should be understood as embodiments and preferred embodiments to any and all aspects of the present invention.

A first study compared the preferred composition of the present invention plus standard antibiotic therapy with placebo plus standard antibiotic therapy in adult patients admitted to intensive care units (ICUs) due to severe community-acquired pneumococcal pneumonia. Two different doses of the preferred composition of the present invention were compared, namely a low dose (4 mg/kg - Low-Dose) and a high does (16 mg/kg - High-Dose). The results showed a synergistic effect of the preferred composition of the present invention (named CAL02) with antibiotic treatment.

As a consequence, the preferred compositions of the present invention, thus, capture and neutralize toxins released from a wide range of bacteria associated with severe infections in a synergistic manner with antibiotic treatment in human patients. The preferred inventive compositions act regardless of the resistance profile of the target pathogen and does not elicit the emergence of resistance.

In a preferred embodiment, said second empty liposome (b) comprises said sphingomyelin as sole lipid component. In a preferred embodiment, said second empty liposome (b) consists of sphingomyelin.

In a preferred embodiment, the amount of cholesterol of said first empty liposome (a) is 30%-70% (weight per weight), and wherein preferably the amount of cholesterol of said first empty liposome (a) is 35%-60% (weight per weight).

In a preferred embodiment, the amount of cholesterol of said empty liposome (a) is 45%-55% (weight per weight), and wherein preferably the amount of cholesterol of said empty liposome (a) is about 50% (weight per weight).

In a preferred embodiment, said first empty liposome (a) consists of cholesterol and sphingomyelin, and wherein preferably the amount of cholesterol of said empty liposome (a) is 45%-55% (weight per weight), and wherein further preferably the amount of cholesterol of said empty liposome (a) is about 50% (weight per weight).

In a preferred embodiment, said first empty liposome (a) comprises said cholesterol and said sphingomyelin as sole lipid components.

In a preferred embodiment, the amount of cholesterol of said empty liposome (a) is 45%-55% (weight per weight), and wherein preferably the amount of cholesterol of said empty liposome (a) is about 50% (weight per weight), and wherein said second empty liposome (b) consists of sphingomyelin.

In a preferred embodiment, said first empty liposome (a) consists of cholesterol and sphingomyelin, and wherein the amount of cholesterol of said empty liposome (a) is 45%-55% (weight per weight), and wherein preferably the amount of cholesterol of said empty liposome (a) is about 50% (weight per weight), and wherein said second empty liposome (b) consists of sphingomyelin.

In a preferred embodiment, said first empty liposome (a) consists of cholesterol and sphingomyelin, and wherein the amount of cholesterol of said empty liposome (a) is 45%-55% (weight per weight), and wherein preferably the amount of cholesterol of said empty liposome (a) is about 50% (weight per weight), and wherein said first empty liposome (a) comprises said cholesterol and said sphingomyelin as sole lipid components, and wherein said second empty liposome (b) consists of sphingomyelin.

In a preferred embodiment, said mixture of empty liposomes comprises at least 20% (weight per weight) of said first (a) and said second (b) empty liposome, and wherein preferably said mixture of empty liposomes comprises at least 30% (weight per weight) of said first (a) and said second (b) empty liposome.

In a preferred embodiment, said mixture of empty liposomes comprises at least 40% (weight per weight) of said first (a) and said second (b) empty liposome.

In a preferred embodiment, said first empty liposome (a) consists of cholesterol and sphingomyelin, and wherein further preferably the amount of cholesterol of said empty liposome (a) is about 50% (weight per weight), and wherein said mixture of empty liposomes comprises at least 40%, preferably at least 45% (weight per weight) of said first (a) and said second (b) empty liposome.

In a preferred embodiment, said first empty liposome (a) consists of a 1:1 (weight per weight -w/w) mixture of said first empty liposomes and said second liposomes, wherein said first empty liposome is composed of a 1:1 weight ratio (1:1 w/w; 35:65 molar ratio) of cholesterol and sphingomyelin, and said second empty liposome is composed exclusively of sphingomyelin.

In a preferred embodiment, said first empty liposome (a) consists of a 1:1 (weight per weight - w/w) mixture of said first empty liposomes and said second liposomes, wherein said first empty liposome is composed of a 1:1 weight ratio (1:1 w/w; 35:65 molar ratio) of cholesterol and sphingomyelin, and said second empty liposome is composed exclusively of sphingomyelin, and wherein said first empty liposome (a) comprises said cholesterol and said sphingomyelin as sole lipid components, and said second empty liposome (b) comprises said sphingomyelin as sole lipid component.

In a preferred embodiment, the mean diameter of said first empty liposome is about 130 nm, and the mean diameter of said second empty liposome is about 90 nm.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably persistent hypotension, in septic shock, wherein said hypotension, preferably said persistent hypotension, is associated with a systolic blood pressure < 90 mm Hg or with mean arterial pressure < 70 mm Hg.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably persistent hypotension, in septic shock, wherein said hypotension, preferably said persistent hypotension, is associated with a systolic blood pressure < 90 mm Hg.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably persistent hypotension, in septic shock, wherein said hypotension, preferably persistent hypotension, is associated with mean arterial pressure < 70 mm Hg.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably persistent hypotension, in septic shock, wherein said hypotension, preferably persistent hypotension, is associated with a systolic blood pressure < 90 mm Hg or with mean arterial pressure < 70 mm Hg, and wherein said hypotension, preferably persistent hypotension, is pre-treated with a vasopressor for at least 2 hours.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably persistent hypotension, in septic shock, wherein said hypotension is associated with a systolic blood pressure < 90 mm Hg or with mean arterial pressure < 70 mm Hg, and wherein said hypotension, preferably said persistent hypotension, is pre-treated with a vasopressor for at least 2 hours after fluid resuscitation.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably said persistent hypotension, in septic shock, wherein said hypotension, preferably said persistent hypotension, is associated with a systolic blood pressure < 90 mm Hg or with mean arterial pressure < 70 mm Hg, and wherein said hypotension, preferably said persistent hypotension, is pre-treated with at least one, preferably one, vasopressor at a therapeutic dose for at least 2 hours.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably said persistent hypotension, in septic shock, wherein said hypotension, preferably said persistent hypotension, is associated with a systolic blood pressure < 90 mm Hg or with mean arterial pressure < 70 mm Hg, and wherein said hypotension, preferably said persistent hypotension, is pre-treated with at least one, preferably one, vasopressor at a therapeutic dose for at least 2 hours, wherein said vasopressor is selected from dopamine, epinephrine, norepinephrine, phenylephrine or vasopressin.

In a preferred embodiment, said composition is for use in the treatment of hypotension, preferably persistent hypotension, in septic shock, sepsis, severe sepsis, acute respiratory distress syndrome or acute lung injury, preferably for use in the treatment of hypotension, preferably said persistent hypotension, in septic shock, wherein said hypotension, preferably said persistent hypotension, is associated with a systolic blood pressure < 90 mm Hg or with mean arterial pressure < 70 mm Hg, and wherein said hypotension, preferably said persistent hypotension, is pre-treated with at least one, preferably one, vasopressor at a therapeutic dose for at least 2 hours, wherein said therapeutic dose of said vasopressor is > 5 mg/kg/min of dopamine or a dose corresponding hereto of said vasopressor, preferably a dose corresponding hereto of epinephrine, norepinephrine, phenylephrine or vasopressin.

In a preferred embodiment, said composition is for use in treatment and prevention of septic shock. In a preferred embodiment, said composition is for use in treatment of hypotension, preferably persistent hypotension. In a preferred embodiment, said hypotension, preferably persistent hypotension, is related to septic shock. In a preferred embodiment, said composition is for use in treatment of persistent hypotension. In a preferred embodiment, said persistent hypotension is related to septic shock.

In a preferred embodiment, said hypotension, preferably persistent hypotension, is associated with a systolic blood pressure < 90 mm Hg (or mean arterial pressure < 70 mm Hg). In a preferred embodiment, said hypotension, preferably said persistent hypotension, is associated with a systolic blood pressure < 90 mm Hg (or mean arterial pressure < 70 mm Hg) despite treatment with vasopressors at therapeutic doses (i.e. dopamine > 5 mg/kg/min or any dose of epinephrine, norepinephrine, phenylephrine or vasopressin) for at least 2 hours after adequate fluid resuscitation.

In a preferred embodiment, said treatment is adjunctive to antibiotic therapy, preferably to standard antibiotic therapy. In a preferred embodiment, said antibiotic of said antibiotic therapy, preferably of said standard antibiotic therapy, is selected from ceftriaxone, spiramycin, amoxicillin, amoxicillin / clavulanic acid, gentamicin, piperacilin / tazobactam, cefuroxime, penicillin, azithromycin, clarithromycin, erythromycin, doxycycline, cefotaxime, ampicillin, Ertapenem, cefepime, imipenem, meropenem, ciprofloxacin, levofloxacin, vancomycin, linezolid, moxifloxacin and gemifloxacin, and wherein preferably said antibiotic of said antibiotic therapy, preferably of said standard antibiotic therapy, is selected from ceftriaxone, spiramycin, amoxicillin, gentamicin, levofloxacine, piperacilin/tazobactam, amoxicillin/clavulanic acid, cefuroxime, and penicillin. In a preferred embodiment, said antibiotic therapy, preferably standard antibiotic therapy, is an intraveneous (IV) or an oral antibiotic therapy, preferably standard antibiotic therapy.

In a preferred embodiment, said human patient has pneumonia, wherein preferably said pneumonia is selected from a Community Acquired Pneumonia (CAP), a Hospitalization Acquired Pneumonia (HAP) and a Ventilator-associated pneumonia (VAP). In a preferred embodiment, said human patient has pneumonia, wherein said pneumonia is a Community Acquired Pneumonia (CAP). In a preferred embodiment, said human patient has pneumonia, wherein said pneumonia is a Hospitalization Acquired Pneumonia (HAP). In a preferred embodiment, said human patient has pneumonia, wherein said pneumonia is a Ventilator-associated pneumonia (VAP). In a preferred embodiment, said human patient has pneumonia, wherein said pneumonia is a severe pneumonia, preferably a severe Community Acquired Pneumonia (sCAP) or a severe Community Acquired Pneumococcal Pneumonia (sCAPP). In a preferred embodiment, said human patient has pneumonia, wherein said pneumonia is a Community Acquired Pneumonia (CAP) or a Community Acquired Pneumococcal Pneumonia (CAPP).

In a preferred embodiment, said human patient has pneumonia, and wherein said pneumonia is caused by *Streptococcus pneumoniae, Staphylococcus aureus, Pseudomonas aeruginosa, Enterococcus faecium, Legionella pneumophilia, Haemophilus influenzae, Klebsiella pneumoniae, Escherichia coli, Acinetobacter baumanii, Bordetella pertussis, Serratia marcescens, Stenotrophomonas maltophilia, Moraxella catarrhalis, or Mycobacterium tuberculosis.* In a preferred embodiment, said human patient has pneumonia, and wherein said pneumonia is a severe pneumonia caused by *Streptococcus pneumoniae, Staphylococcus aureus, Pseudomonas aeruginosa, Enterococcus faecium, Legionella pneumophilia, Haemophilus influenzae, Klebsiella pneumoniae, Escherichia coli, Acinetobacter baumanii, Bordetella pertussis, Serratia marcescens, Stenotrophomonas maltophilia, Moraxella catarrhalis, or Mycobacterium tuberculosis,* and wherein preferably said pneumonia, preferably said severe pneumonia is caused by *Streptococcus pneumoniae, Staphylococcus aureus, Pseudomonas aeruginosa, Enterococcus faecium, Haemophilus influenzae, Klebsiella pneumoniae, Escherichia coli, Acinetobacter baumanii, Bordetella pertussis, Moraxella catarrhalis, of Mycobacterium tuberculosis,* and wherein further preferably said pneumonia, preferably said severe Pneumonia is caused by *Streptococcus pneumoniae, Staphylococcus aureus, Klebsiella pneumoniae,* or *Pseudomonas aeruginosa,* and wherein again further preferably said pneumonia or said severe pneumonia is caused by *Streptococcus pneumoniae.*

In a preferred embodiment, said composition is in the form of a solution for intravenous administration, preferably intravenous infusion, comprising between 10 and 40 gram of said mixture of empty liposomes per liter solution, preferably between 10 and 20 gram of said mixture of empty liposomes per liter solution.

In a preferred embodiment, the infusion time of said intravenous administration is up to 3 hours, and wherein preferably the infusion time of said intravenous administration is 10 minutes to 2 hours.

In a preferred embodiment, said composition is administered in at least 2 doses. In a preferred embodiment, said composition is administered in at least 2 doses, in a first dose and in a second dose, and wherein the interval between said first dose and said second dose is 6 to 96 hours, preferably 12 to 72 hours, further preferably 24 to 48 hours and again further preferably 24 or 48 hours.

In a preferred embodiment, said composition is administered in 2 to 4 doses, preferably in two doses, over 12 to 72 hours, further preferably in two doses over 24 to 48 hours, preferably with an interval of 24 or 48 hours.

In a preferred embodiment, said composition is administered in at least 2 doses to said human patient, in a first dose and in a second dose, and wherein the interval between said first dose and said second dose is 20 to 28 hours, preferably 24 hours.

In a preferred embodiment, said composition is administered to said human patient in 2 doses, in a first dose and in a second dose, wherein an interval of 20 to 28 hours, preferably of 24 hours is between said administration of said first dose and said administration of said second dose.

In a preferred embodiment, each of said dose is 1mg/kg to 64mg/kg, preferably 2mg/kg to 32mg/kg, further preferably 3 to 25 mg/kg, again further preferably 4mg/kg to 16mg/mg.

In a preferred embodiment, each of said dose is 2mg/kg to 8mg/kg, preferably 2mg/kg to 6mg/kg, further preferably 3 to 5 mg/kg, again further preferably 4mg/kg.

In a preferred embodiment, each of said dose is 10mg/kg to 22/kg, preferably 12mg/kg to 20/kg, further preferably 14mg/kg to 18/kg, again further preferably 16 mg/kg.

In a preferred embodiment, said composition is administered in the form of a solution for intravenous administration.

In a preferred embodiment, said composition is administered to said human patient in, preferably at least, 2 doses, in a first dose and in a second dose, wherein an interval of 20 to 48 hours, preferably of 24 hours is between said administration of said first dose and said administration of said second dose, and wherein said composition is in the form of a solution for intravenous administration.

In a preferred embodiment, said composition is for use in the treatment of sepsis, severe sepsis, septic shock, or prolonged and severe hypotension preferably persistent hypotension, and wherein preferably said composition is for use in the treatment of septic shock in an animal, preferably in a human, and wherein said sepsis, severe sepsis, septic shock, or prolonged and severe hypotension preferably persistent hypotension, preferably septic shock, requires stay at the hospital.

In a preferred embodiment, said composition is for use in the treatment of sepsis, septic shock, or hypotension, preferably persistent hypotension, in an animal, preferably in a human. In a preferred embodiment, said composition is for use in the treatment of sepsis, septic shock, or hypotension, preferably persistent hypotension, in a human. In a very preferred embodiment, said composition is for use in the treatment of sepsis in a human. In a very preferred embodiment, said composition is for use in the treatment of septic shock in a human. In a very preferred embodiment, said composition is for use in the treatment of hypotension, preferably persistent hypotension, in a human. In a preferred embodiment, said sepsis, septic shock, or said hypotension, preferably persistent hypotension, requires the stay of said human at the hospital, preferably at the intensive care unit (ICU) in a hospital.

In a preferred embodiment, said composition is for use in the treatment of sepsis, severe sepsis, septic shock, or prolonged and severe hypotension preferably persistent hypotension, and wherein preferably said composition is for use in the treatment of septic shock in an animal, preferably in a human, and wherein said sepsis, severe sepsis, septic shock, or prolonged and severe hypotension preferably persistent hypotension, preferably septic shock, requires stay at the intensive care unit (ICU), preferably at the intensive care unit (ICU) in a hospital.

In a preferred embodiment, said composition is for use in the treatment of sepsis, severe sepsis, septic shock, or prolonged and severe hypotension preferably persistent hypotension, and wherein preferably said composition is for use in the treatment of septic shock in an animal, preferably in a human, and wherein said sepsis, severe sepsis, septic shock, or prolonged and severe hypotension preferably persistent hypotension, preferably septic shock, requires stay at the hospital and wherein said treatment reduces the duration of stay at said hospital as compared to a stay at the hospital when no such treatment is effected.

In a preferred embodiment, the reduction of duration of stay at the hospital due to said treatment is at least one day, preferably two days, further preferably three days, again further preferably four days, again further preferably five days, again further preferably six days, again further preferably seven days, again further preferably eight days, again further preferably nine days. In a preferred embodiment, the duration of stay at the hospital is at most 18 days.

In a preferred embodiment, said composition is for use in the treatment of sepsis, severe sepsis, septic shock, or prolonged and severe hypotension preferably persistent hypotension, and wherein preferably said composition is for use in the treatment of septic shock in an animal, preferably in a human, and wherein said sepsis, severe sepsis, septic shock, or prolonged and severe hypotension preferably persistent hypotension, preferably septic shock, requires stay at the intensive care unit (ICU), preferably at the intensive care unit (ICU) in a hospital. and wherein said treatment reduces the duration of stay at the intensive care unit (ICU) as compared to a stay at the intensive care unit (ICU) when no such treatment is effected.

In a preferred embodiment, the reduction of duration of stay at the intensive care unit (ICU) is at least one day, preferably two days, further preferably three days, again further preferably four days, again further preferably five days, again further preferably six days, again further preferably seven days. In a preferred embodiment, the duration of stay at the intensive care unit (ICU) is at most 18 days.

In a preferred embodiment, said composition is for use in the treatment of sepsis, severe sepsis, septic shock, or prolonged and severe hypotension preferably persistent hypotension, and wherein preferably said composition is for use in the treatment of septic shock in an animal, preferably in a human, and wherein said sepsis, severe sepsis, septic shock, or prolonged and severe hypotension preferably persistent hypotension, preferably septic shock, is cured in less time as compared when no such treatment is effected.

In a preferred embodiment, said cure in less time is at least one day less in time, preferably two days less in time or further preferably at least three days less in time, again further preferably at least four days, again further preferably at least five days, again further preferably at least six days, again further preferably at least seven days less in time.

In a preferred embodiment, said composition is for use in the treatment of sepsis, severe sepsis, septic shock, or prolonged and severe hypotension preferably persistent hypotension, and wherein preferably said composition is for use in the treatment of septic shock in an animal, preferably in a human, and wherein said treatment decreases the Cardiovascular SOFA score as compared to said Cardiovascular SOFA when no such treatment is effected.

In a preferred embodiment, said composition is for use in the treatment of sepsis, severe sepsis, septic shock, or prolonged and severe hypotension, preferably persistent hypotension, and wherein preferably said composition is for use in the treatment of septic shock in an animal, preferably in a human, and wherein said treatment decreases the Cardiovascular SOFA score as compared to said Cardiovascular SOFA when no such treatment is effected, and wherein said decrease is at least 50%, preferably at least 60%, further preferably at least 70%, again further preferably at least 80%, after 7 days of the start of said treatment, preferably after 6 days of the start of said treatment, further preferably after 5 days.

The invention furthermore comprises the following items:
1. A composition comprising, preferably consisting of, a mixture of empty liposomes, wherein said mixture of empty liposomes comprises, preferably consists of,
   (a) a first empty liposome comprising cholesterol, wherein the amount of cholesterol is at least 30% (weight per weight); and
   (b) a second empty liposome comprising sphingomyelin, wherein preferably said second empty liposome (b) comprises said sphingomyelin as sole lipid component;
   for use in the treatment of sepsis in an animal, preferably in a human.
2. The composition for use of item 1, wherein said composition is for use in the treatment of septic shock in said animal, preferably in said human.
3. The composition for use of item 1, wherein said composition is for use in the treatment of hypotension, preferably persistent hypotension, of said animal, preferably said human, with sepsis.
4. The composition for use of item 2, wherein said composition is for use in the treatment of hypotension, preferably persistent hypotension, of said animal, preferably said human with septic shock.
5. The composition for use of any one of the preceding items, wherein the amount of cholesterol of said empty liposome (a) is 45%-55% (weight per weight), and wherein said second empty liposome (b) consists of sphingomyelin.
6. The composition for use of any one of the preceding items, wherein said first empty liposome (a) consists of cholesterol and sphingomyelin, and wherein the amount of cholesterol of said empty liposome (a) is about 50% (weight per weight), and wherein said mixture of empty liposomes comprises at least 40%, preferably at least 45% (weight per weight) of said first (a) and said second (b) empty liposome.
7. The composition for use of any one of the preceding items, wherein said first empty liposome (a) consists of a 1:1 (weight per weight - w/w) mixture of said first empty liposomes and said second liposomes, wherein said first empty liposome is composed of a 1:1 weight ratio (1:1 w/w; 35:65 molar ratio) of cholesterol and sphingomyelin, and said second empty liposome is composed exclusively of sphingomyelin, and wherein said first empty liposome (a) comprises said cholesterol and said sphingomyelin as sole lipid components, and said second empty liposome (b) comprises said sphingomyelin as sole lipid component.
8. The composition for use of any one of the items 3 to 7, wherein said hypotension, preferably said persistent hypotension, is associated with mean arterial pressure < 70 mm Hg.
9. The composition for use of item 8, wherein said hypotension, preferably said persistent hypotension, is pre-treated with a vasopressor for at least 2 hours.
10. The composition for use of any one of the preceding items, wherein said treatment is adjunctive to antibiotic therapy.
11. The composition for use of item 10, wherein said antibiotic therapy is an intraveneous (IV) or an oral antibiotic therapy.
12. The composition for use of any one of the preceding items, wherein said animal is a human patient, and wherein said human patient has pneumonia, wherein preferably said pneumonia is selected from a Community Acquired Pneumonia (CAP), a Hospitalization Acquired Pneumonia (HAP) and a Ventilator-associated pneumonia (VAP).
13. The composition for use of item 12, wherein said pneumonia is a severe pneumonia, preferably a severe Community Acquired Pneumonia (sCAP) or a severe Community Acquired Pneumococcal Pneumonia (sCAPP).
14. The composition for use of items 12 or 13, wherein said pneumonia or said severe pneumonia is caused by *Streptococcus pneumoniae, Staphylococcus aureus, Pseudomonas aeruginosa, Enterococcus faecium, Legionella pneumophilia, Haemophilus influenzae, Klebsiella pneumoniae, Escherichia coli, Acinetobacter baumanii, Bordetella pertussis, Serratia marcescens, Stenotrophomonas maltophilia, Moraxella catarrhalis, or Mycobacterium tuberculosis,* wherein preferably said pneumonia or said severe pneumonia is caused by *Streptococcus pneumoniae.*
15. The composition for use of any one of the preceding items, wherein said composition is in the form of a solution for intravenous administration.
16. The composition for use of any one of the preceding items, wherein said composition is administered to said animal, preferably to said human, in at least 2 doses, in a first dose and in a second dose, and wherein the interval between said first dose and said second dose is 6 to 96 hours, preferably 12 to 72 hours, further preferably 24 to 48 hours and again further preferably 24 or 48 hours.
17. The composition for use of any one of the preceding items, wherein said sepsis, septic shock, or said hypotension, preferably persistent hypotension, requires stay at the hospital, preferably at the intensive care unit (ICU) in a hospital.
18. The composition for use of item 17, wherein said treatment reduces the duration of stay at said hospital as compared to a stay at the hospital when no such treatment is effected.
19. The composition for use of item 17 or item 18, wherein the reduction of duration of stay at the hospital due to said treatment is at least one day, preferably two days, further preferably three days, again further preferably four days, again further preferably five days, again further preferably six days, again further preferably seven days, again further preferably eight days, again further preferably nine days.
20. The composition for use of any one of the preceding items, wherein said sepsis, septic shock, or said hypotension, preferably persistent hypotension, is cured in less time as compared when no such treatment is effected.
21. The composition for use of item 20, wherein said cure in less time is at least one day less in time, preferably two days less in time or further preferably at least three days less in time, again further preferably at least four days, again further preferably at least five days, again further preferably at least six days, again further preferably at least seven days less in time.
22. The composition for use of any one of the preceding items, wherein said treatment decreases the Cardiovascular SOFA score as compared to said Cardiovascular SOFA when no such treatment is effected.
23. The composition for use of item 22, wherein said decrease is at least 50%, preferably at least 60%, further preferably at least 70%, again further preferably at least 80%, after 7 days of the start of said treatment, preferably after 6 days of the start of said treatment, further preferably after 5 days.

### EXAMPLES

### Liposomes:

Sphingomyelin (CAS No. 85187-10-6) from egg yolk was purchased from Sigma (S0756), Avanti Polar Lipids (860061) or Lipoid GmbH. Cholesterol (CAS No. 57-88-5) from ovine wool grease was purchased from Sigma (C-8667), Avanti Polar Lipids (70000) or Dishman Netherlands B.V.. It is in accordance with the present invention that sphingomyelin and cholesterol comprised by or consisted of by the inventive mixtures of empty liposomes may be either obtained from natural sources as indicated above or alternatively by way of chemical synthesis.

### Liposomes preparation:

Unilamellar sphingomyelin: cholesterol (35:65 molar ratio) and sphingomyelin only (100%) liposomes were prepared using sonication or microfluidization (e.g. high pressure homogenization or according to a hydration, extrusion, and diafiltration process protocol.

### Sonication:

The lipids were individually dissolved in chloroform at 1 mg/ml concentrations and stored at -20°C. For the preparation of liposomes the chloroform solutions of individual lipids were mixed in the proportions, as required, to produce routinely 50-500 µl of the final solution. Chloroform was completely evaporated for 20-50 min at 60°C. 50 µl or 100 µl of Tyrode's buffer (140 mM NaCl, 5 mM KCl, 1 mM MgCl₂, 10 mM glucose, 10 mM HEPES; pH=7.4) containing 2.5 mM CaCl₂ was added to the tubes containing films of dried lipids and vigorously vortexed. The lipid suspensions were incubated for 20-30 min at 45°C in an Eppendorf thermomixer with vigorous shaking. To produce liposomes, the final lipid suspensions were sonicated 3x5 sec at 6°C in a Bandelin Sonopuls sonicator at 70% power. The liposomal preparations were left for at least 1 hour at 6°C before they were used in experiments.

### Hydration, extrusion, and diafiltration process protocol:

In an alternative method, each liposomal formulation was made by the ethanol hydration and extrusion method. Lipids were individually dissolved in ethanol and t-butanol while mixing at elevated temperature (~55°C). The lipid solution was then added to a PBS Buffer Solution (Sodium Chloride, Monosodium Phosphate, Dihydrate and Disodium Phosphate, Dihydrate dissolved in water for injection while mixing, adjusted to a pH of 7.0-7.4 with either hydrochloric acid (HCl) or sodium hydroxide (NaOH) as required, and filtered through a 0.2 µm filter) while mixing at elevated temperature (~65°C) for approximately 30 minutes. The ensuing process fluid was then extruded repeatedly through a series of polycarbonate track-etched membranes at elevated pressure and temperature (~65°C) until the desired particle size was achieved, as measured by dynamic light scattering (example of extruder: LIPEX^{®} Extruders). The ensuing process fluid was then concentrated approximately 2-fold using a 100,000 molecular weight cut-off hollow-fiber cartridge and then diafiltered against approximately 10 volume exchanges of the PBS Buffer Solution to remove the ethanol and t-butanol. At the end of diafiltration, the process fluid was concentrated by approximately 30% to allow subsequent dilution to target lipid concentration. Before dilution, the process fluid was filtered through a 0.2 µm sterilizing-grade filter in order to remove any larger liposomes which could clog the filter during Sterile Filtration. The process fluid was then diluted to a target of 40 mg/mL total lipid with the PBS Buffer Solution. The final formulations were aseptically filtered through two 0.2 µm sterilizing grade filters in series and aseptically filled into glass tubing vials.

The concentration of individual lipids in the liposomes is always given as the weight per weight ratio. In liposomes containing sphingomyelin and cholesterol, the 1:1 (weight per weight) ratio corresponds to 50% (weight per weight) or to 35:65 molar ratio. Specifications are described in Table 1.

**Table 1: Specifications liposomes**

| Mean diameter (nm) | Polydispersity index | Zeta potential (mV) | Osmolality (mmol/kg) | pH |
|---|---|---|---|---|
| 40 to 400 | < 0.45 | -25 to +2 | 250-400 | 6.5-8.0 |

### CURB-65 Score:

CURB-65, also known as the CURB criteria, is a clinical prediction rule well-known to the skilled person in the art that has been validated for predicting mortality in community-acquired pneumonia (Lim WS, et al. (2003) Thorax 58(5): 377-82). The CURB-65 is recommended by the British Thoracic Society for the assessment of severity of pneumonia (British Thoracic Society Standards of Care Committee (2001). "BTS Guidelines for the Management of Community Acquired Pneumonia in Adults". Thorax. 56. Suppl 4: IV1-64)

The score is an acronym for each of the risk factors measured. Each risk factor scores one point, for a maximum score of 5:
- Confusion of new onset (defined as an AMTS of 8 or less)
- Blood **U**rea nitrogen greater than 7 mmol/l (19 mg/dL)
- **R**espiratory rate of 30 breaths per minute or greater
- **B**lood pressure less than 90 mmHg systolic or diastolic blood pressure 60 mmHg or less
- Age **65** or older.

### APACHE II:

The APACHE II is an acronym for **A**cute **P**hysiology **a**nd **C**hronic **H**ealth **E**valuation and is calculated according to Table 2 (Knaus WA et al. APACHE II: a severity of disease classification system. Crit Care Med. 1985 Oct;13(10):818-29):

**Table 2: APACHE II Score**

| **Age** | **POINTS** |
|---|---|
| under 45 | 0 |
| 45-54 | 2 |
| 55-64 | 3 |
| 65-74 | 5 |
| over 74 | 6 |

| **History of severe organ insufficiency or immunocompromised** | |
|---|---|
| Yes, and non-operative or emergency post-operative patient | 5 |
| Yes, and elective post-operative patient | 2 |
| No | 0 |

| **Temperature °C** | |
|---|---|
| over 40.9 | 4 |
| 39-40.9 | 3 |
| 38.5-38.9 | 1 |
| 36-38.4 | 0 |
| 34-35.9 | 1 |
| 32-33.9 | 2 |
| 30-31.9 | 3 |
| below 30 | 4 |

| **Mean arterial pressure (mm Hg)** | |
|---|---|
| over 159 | 4 |
| 130-159 | 3 |
| 110-129 | 2 |
| 70-109 | 0 |
| 50-69 | 2 |
| below 50 | 4 |

| **Heart rate (ventricular response)** | |
|---|---|
| over 179 | 4 |
| 140-179 | 3 |
| 110-139 | 2 |
| 70-109 | 0 |
| 55-69 | 2 |
| 40-54 | 3 |
| below 40 | 4 |

| **Respiratory Rate (non-ventilated or ventilated** | |
|---|---|
| over 49 | 4 |
| 35-49 | 3 |
| 25-34 | 1 |
| 12-24 | 0 |
| 10-11 | 1 |
| 6-9 | 2 |
| below 6 | 4 |

| **Oxygenation (use PaO2 if FiO2 <50%, otherwise A-a** | |
|---|---|
| A-a gradient over 499 | 4 |
| A-a gradient 350-499 | 3 |
| A-a gradient 200-349 | 2 |
| A-a below 200 (if FiO2 over 49%) or pO2 more than 70 (if | 0 |
| pO2 = 61-70 | 1 |
| pO2 = 55-60 | 3 |
| pO2 below 55 | 4 |

| **Arterial pH** | |
|---|---|
| over 7.69 | 4 |
| 7.60-7.69 | 3 |
| 7.50-7.59 | 1 |
| 7.33-7.49 | 0 |
| 7.25-7.32 | 2 |
| 7.15-7.24 | 3 |
| below 7.15 | 4 |

| **Serum sodium (mmol/L)** | |
|---|---|
| over 179 | 4 |
| 160-179 | 3 |
| 155-159 | 2 |
| 150-154 | 1 |
| 130-149 | 0 |
| 120-129 | 2 |
| 111-119 | 3 |
| below 111 | 4 |

| **Serum potassium (mmol/L)** | |
|---|---|
| over 6.9 | 4 |
| 6-6.9 | 3 |
| 5.5-5.9 | 1 |
| 3.5-5.4 | 0 |
| 3-3.4 | 1 |
| 2.5-2.9 | 2 |
| below 2.5 | 4 |

| **Serum creatinine (mg/ dL)** | |
|---|---|
| over 3.4 and ACUTE renal failure | 8 |
| 2.0-3.4 and ACUTE renal failure | 6 |
| over 3.4 and chronic | 4 |
| 1.5-1.9 and ACUTE renal failure | 4 |
| 2.0-3.4 and chronic | 3 |
| 1.5-1.9 | 2 |
| 0.6-1.4 | 0 |
| below 0.6 | 2 |

| **Hematocrit (%)** | |
|---|---|
| over 59.9 | 4 |
| 50-59.9 | 2 |
| 46-49.9 | 1 |
| 30-45.9 | 0 |
| 20-29.9 | 2 |
| below 20 | 4 |

| **White blood count (total/cubic mm in 1000's)** | |
|---|---|
| over 39.9 | 4 |
| 20-39.9 | 2 |
| 15-19.9 | 1 |
| 3.0-14.9 | 0 |
| 1.0-2.9 | 2 |
| below 1.0 | 4 |

| **Glasgow Coma scale** | |
|---|---|
| **Eyes open** | |
| Spontaneous | 4 |
| To speech | 3 |
| To pain | 2 |
| Absent | 1 |

| **Verbal** | |
|---|---|
| Converses / Oriented | 5 |
| Converses / Disoriented | 4 |
| Inappropriate | 3 |
| Incomprehensible | 2 |
| Absent | 1 |

| **Motor** | |
|---|---|
| Obeys | 6 |
| Localises pain | 5 |
| Withdraws (flexion) | 4 |
| Decorticate (flexion) rigidity | 3 |
| Decerebrate (flexion) rigidity | 2 |
| Absent | 1 |
| **Score relative to GCS: 15 - (minus) Actual GCS** | |

The APACHE II Score aims at providing an approximate of the chances of mortality of a patient or a group of patients based on the total score obtained. The mortality interpretation of the score is described in Table 3:

**Table 3: Apache II Score / Approximate Mortality Interpretation**

| Total points range | Chance of mortality if patient had no operation | Chance of mortality in post-operative situation |
|---|---|---|
| 0-4 | 4% | 1% |
| 5-9 | 8% | 3% |
| 10-14 | 15% | 7% |
| 15-19 | 24% | 12% |
| 20-24 | 40% | 30% |
| 25-29 | 55% | 35% |
| 30-34 | 73% | 73% |
| 35-100 | 85% | 88% |

### SOFA:

The SOFA score is an acronym for **S**equential **O**rgan **F**ailure **A**ssessment and is calculated according to Table 4 (S. Vosylius, J. Sipylaite and J. Ivaskevicius, Croat Med J, 45 (2004), 715-20):

**Table 4: SOFA Score**

| | **Score** | | | | |
|---|---|---|---|---|---|
| **Variables** | **0** | **1** | **2** | **3** | **4** |
| **Respiration: PaO2/FiO2** | >400 | 301-400 | 201-300 | 101-200* | ≤100* |
| **Coagulation: platelets, 10³/µl** | >150 | 101-150 | 51-100 | 21-50 | ≤20 |
| **Liver: bilirubin, µmol/L** | <20 | 20-32 | 33-101 | 102-204 | >204 |
| **Cardiovascular: hypotension** | No hypotension | MAP**<70 mm Hg | Dopamine≤5, or dobutamine (any dose)*** | Dopamine>5, or epinephrine≤0.1, or norepinephrine≤0.1** * | Dopamine>15, or epinephrine>0.1, or norepinephrine>0.1*** |
| **Central Nervous system:** | 15 | 13-14 | 10-12 | 6-9 | <6 |
| **Glasgow Coma Score ****** | | | | | |
| **Renal:** | <110 | 110-170 | 171-299 | 300-440 | <440 |
| **Creatinine µmol/L** | | | | | |
| * With respiratory support | | | | | |
| ** MAP - mean arterial pressure | | | | | |
| *** Adrenergic agents administered for at least 1h (dosages are in µg/kg/min) | | | | | |
| **** As defined in the APACHE II Score above | | | | | |

### Cardiovascular hypotension:

Cardiovascular hypotension is a predominant feature of septic shock. It may be caused by either low cardiac output or low systemic vascular resistance. Its severity may be assessed using a Cardiovascular SOFA (Sequential Organ Failure Assessment) score, defined by the mean arterial pressure or by the need to administer vasopressors, as described in Table 5 (S. Vosylius, J. Sipylaite and J. Ivaskevicius, Croat Med J, 45 (2004), 715-20):

**Table 5: Cardiovascular SOFA Score**

| **Mean arterial pressure OR administration of vasopressors required** | **Score** |
|---|---|
| No hypotension | 0 |
| MAP < 70 mm/Hg | 1 |
| dopamine ≤ 5 µg/kg/min or dobutamine (any dose) | 2 |
| dopamine > 5 µg/kg/min OR epinephrine ≤ 0.1 µg/kg/min OR norepinephrine ≤ 0.1 µg/kg/min | 3 |
| dopamine > 15 µg/kg/min OR epinephrine > 0.1 µg/kg/min OR norepinephrine > 0.1 µg/kg/min | 4 |

### EXAMPLE 1

### Treatment of sepsis and septic shock in severely-infected patients

In the study reported thereafter, a very preferred mixture of empty liposomes of the present invention, named CAL02, is administered intravenously (IV) as adjunctive treatment in addition to standard antibiotic therapy, in patients admitted to the intensive care unit (ICU) due to a severe community-acquired pneumonia (CAP) caused by *Streptococcus pneumoniae.* Said very preferred inventive mixture of empty liposomes (CAL02) consists of a 1:1 (weight per weight - w/w) mixture of said first empty liposomes and said second liposomes, wherein said first empty liposome is composed of a 1:1 weight ratio (1:1 w/w; 35:65 molar ratio) of sphingomyelin and cholesterol, and said second empty liposomes is composed exclusively of sphingomyelin.

Beside standard-of-care antibiotic therapy (Mandell et al, CID 2007:44 (Suppl 2), S27-S72) each patient received two infusions of CAL02 or placebo (physiological 0.9% NaCl solution) (with a 24-hour or 48-hour interval between these administrations). The first administration was given shortly after the diagnosis of severity. Diagnosis of severity was based on at least one of the following severity criteria:
i. invasive mechanical ventilation support
ii. treatment with vasopressors at therapeutic doses (i.e. dopamine > 5 mg/kg/min or any dose of epinephrine, norepinephrine, phenylephrine or vasopressin) for at least 2 hours to maintain or attempt to maintain systolic blood pressure > 90 mm

Hg (or mean arterial pressure > 70 mm Hg) after adequate fluid resuscitation or on at least three of the following minor severity criteria:
i. Respiratory rate ≥ 30 breaths/min
ii. PaO2/FiO2 ratio ≤ 250 mm Hg
iii. Multilobar infiltrates
iv. Confusion/disorientation (has to be documented prior to the use of sedative or other new psychotropic medication)
v. Urea > 7 mM (> 40 mg/dL)
vi. Leukopenia (white blood cell count < 4'000 cells/mm³)
vii. Thrombocytopenia (platelet count < 100'000 cells/mm³)
viii. Hypothermia (core temperature < 36°C)
ix. Systolic blood pressure < 90 mm Hg or mean arterial pressure < 70 mm Hg and received ≥ 40 mL/kg of fluid resuscitation over at least 2 hours

Two dose levels of CAL02 (4 mg/kg (Low Dose) and 16 mg/kg (High Dose)) were tested. Five patients were randomized to the Placebo arm, 11 to the CAL02 High Dose arm, and 3 to the CAL02 Low Dose arm.

At the time of treatment, 56% of patients were already in septic shock: 2 patients in the Placebo arm, 5 in the CAL02 High Dose arm, and all three patients in the CAL02 Low Dose arm.

### Study results

**Table 6: Patients characteristics at baseline**

| | | | PLACEBO (n = 5) | CAL02 HIGH DOSE (n=10) | CAL02 LOW DOSE (n = 3) | CAL02 Combined (n=13) |
|---|---|---|---|---|---|---|
| Demographic characteristics | | | | | | |
| | Age | Mean | 62.2 | 57.5 | 50.7 | 55.9 |
| | BMI | Mean | 29.1 | 25.9 | 20.9 | 24.7 |
| Clinical characteristics | | | | | | |
| | CURB-65 | Mean | 3.2 | 3.5 | 3.3 | 3.5 |
| | APACHE II | Mean | 17.4 | 22.1 | 25.3 | 22.8 |
| | SOFA | Mean | 7 | 6.5 | 11.0 | 7.5 |
| | Septic shock (blood pressure < 90 mm Hg (or mean arterial pressure < 70 mm Hg) despite treatment with therapeutic dose of vasopressors) | n | 2 | 5 | 3 | 9 |
| | Mechanical ventilation | n | 2 | 2 | 3 | 5 |
| | Bacteremia | n | 0 | 3 | 2 | 5 |
| | Severe CAP criteria | Major | 3 | 6 | 3 | 9 |
| | | Minor | 2 | 4 | 0 | 4 |
| | Charlson commorbities index | Mean | 2.8 | 3.4 | 5.3 | 3.8 |
| | Renal failure | n | 2 | 4 | 2 | 6 |
| | Cardiac disorders | n | 3 | 5 | 0 | 5 |
| | Thrombocytopenia | n | 1 | 3 | 2 | 5 |
| | PaO2/FiO2 ratio | Median | 113 | 141.5 | 73 | 110 |
| | CRP | Median | 376 | 276.35 | 314 | 303 |
| | Procalcitonin | Median | 7.04 | 16.3 | 20 | 20 |

A faster decrease in the Cardiovascular SOFA score was observed in the CAL02 arms as compared to the Placebo arm, both when the entire study population was considered and when only patients presenting a septic shock at baseline were assessed: A 100% decrease in the SOFA score was already achieved in 6 days in the CAL02 arms while the decrease in the placebo arm did not reach 40% at the same time point (Figures 1 and 2).

Among the patients presenting a septic shock, all patients (5/5, 100%) in the CAL02 High Dose arm and 66% (2/3) of the patients in the CAL02 Low Dose arm, as compared to no patient (0/2, 0%) in the placebo group had a complete resolution of hypotension and of septic shock at Day 8 after treatment.

Of the 3 patients in the placebo arm that were not in septic shock at baseline, one was hypotensive at baseline and developed a septic shock at Day 4, and two had no hypotension at baseline but developed hypotension within the first 8 days. In contrast, in the CAL02 arms, 3 patients were not hypotensive at baseline and did not develop any hypotension and all the others improved to resolution before day 6. This suggests that CAL02 prevented hypotension and hemodynamic instability and protected against the development of septic shock.

The resolution of the septic shock was accompanied by strong reduction of the mean ICU stay, from 32 days for the patients in the placebo arm to 5.4 and 15 for the patients in the CAL02 High Dose arm and CAL02 Low Dose arm, respectively. Furthermore there was a lower incidence of mortality in the CAL02 arms (20% and 33% mortality in the CAL02 High dose arm and the CAL02 Low Dose arm, respectively) as compared to the placebo arm (50%) (Table 7).

**Table 7: Outcome**

| | Placebo (n = 2) | CAL02 High Dose (n = 5) |
|---|---|---|
| Mortality (n) | 1 (50%) | 1 (20%) |
| Resolution of the hypotension at Day 8 (n, %) | 0 (0%) | 5 (100%) |
| Time to ICU discharge (mean) | 32 | 5.4 |

The impact of CAL02 treatment was also evaluated on vital signs (including include heart rate, systolic and diastolic blood pressure and core body temperature) as well as on lactate levels.

## Claims

1. A composition comprising, preferably consisting of, a mixture of empty liposomes, wherein said mixture of empty liposomes comprises, preferably consists of,
(a) a first empty liposome comprising cholesterol, wherein the amount of cholesterol is at least 30% (weight per weight); and
(b) a second empty liposome comprising sphingomyelin, wherein preferably said second empty liposome (b) comprises said sphingomyelin as sole lipid component;
for use in the treatment of sepsis in an animal, preferably in a human.

2. The composition for use of claim 1, wherein said composition is for use in the treatment of septic shock in said animal, preferably in said human.

3. The composition for use of claim 1, wherein said composition is for use in the treatment of hypotension, preferably persistent hypotension, of said animal, preferably said human, with sepsis.

4. The composition for use of claim 2, wherein said composition is for use in the treatment of hypotension, preferably persistent hypotension, of said animal, preferably said human with septic shock.

5. The composition for use of any one of the preceding claims, wherein the amount of cholesterol of said empty liposome (a) is 45%-55% (weight per weight), and wherein said second empty liposome (b) consists of sphingomyelin.

6. The composition for use of any one of the preceding claims, wherein said first empty liposome (a) consists of cholesterol and sphingomyelin, and wherein the amount of cholesterol of said empty liposome (a) is about 50% (weight per weight), and wherein said mixture of empty liposomes comprises at least 40%, preferably at least 45% (weight per weight) of said first (a) and said second (b) empty liposome.

7. The composition for use of any one of the preceding claims, wherein said first empty liposome (a) consists of a 1:1 (weight per weight - w/w) mixture of said first empty liposomes and said second liposomes, wherein said first empty liposome is composed of a 1:1 weight ratio (1:1 w/w; 35:65 molar ratio) of cholesterol and sphingomyelin, and said second empty liposome is composed exclusively of sphingomyelin, and wherein said first empty liposome (a) comprises said cholesterol and said sphingomyelin as sole lipid components, and said second empty liposome (b) comprises said sphingomyelin as sole lipid component.

8. The composition for use of any one of the claims 3 to 7, wherein said hypotension, preferably said persistent hypotension, is associated with mean arterial pressure < 70 mm Hg, wherein further preferably said hypotension, preferably said persistent hypotension, is pre-treated with a vasopressor for at least 2 hours..

9. The composition for use of any one of the preceding claims, wherein said treatment is adjunctive to antibiotic therapy, wherein preferably said antibiotic therapy is an intraveneous (IV) or an oral antibiotic therapy.

10. The composition for use of any one of the preceding claims, wherein said animal is a human patient, and wherein said human patient has pneumonia, wherein preferably said pneumonia is selected from a Community Acquired Pneumonia (CAP), a Hospitalization Acquired Pneumonia (HAP) and a Ventilator-associated pneumonia (VAP), wherein further preferably said pneumonia is a severe pneumonia, preferably a severe Community Acquired Pneumonia (sCAP) or a severe Community Acquired Pneumococcal Pneumonia (sCAPP).

11. The composition for use of claim 10, wherein said pneumonia or said severe pneumonia is caused by *Streptococcus pneumoniae, Staphylococcus aureus, Pseudomonas aeruginosa, Enterococcus faecium, Legionella pneumophilia, Haemophilus influenzae, Klebsiella pneumoniae, Escherichia coli, Acinetobacter baumanii, Bordetella pertussis, Serratia marcescens, Stenotrophomonas maltophilia, Moraxella catarrhalis, or Mycobacterium tuberculosis,* wherein preferably said pneumonia or said severe pneumonia is caused by *Streptococcus pneumoniae.*

12. The composition for use of any one of the preceding claims, wherein said composition is in the form of a solution for intravenous administration.

13. The composition for use of any one of the preceding claims, wherein said composition is administered to said animal, preferably to said human, in at least 2 doses, in a first dose and in a second dose, and wherein the interval between said first dose and said second dose is 6 to 96 hours, preferably 12 to 72 hours, further preferably 24 to 48 hours and again further preferably 24 or 48 hours.

14. The composition for use of any one of the preceding claims, wherein said sepsis, septic shock, or said hypotension, preferably persistent hypotension, requires stay at the hospital, preferably at the intensive care unit (ICU) in a hospital, wherein preferably said treatment reduces the duration of stay at said hospital as compared to a stay at the hospital when no such treatment is effected, and wherein further preferably wherein the reduction of duration of stay at the hospital due to said treatment is at least one day, preferably two days, further preferably three days, again further preferably four days, again further preferably five days, again further preferably six days, again further preferably seven days, again further preferably eight days, again further preferably nine days.

15. The composition for use of any one of the preceding claims, wherein said treatment decreases the Cardiovascular SOFA score as compared to said Cardiovascular SOFA when no such treatment is effected, wherein preferably said decrease is at least 50%, further preferably at least 60%, further preferably at least 70%, again further preferably at least 80%, after 7 days of the start of said treatment, preferably after 6 days of the start of said treatment, further preferably after 5 days.
